(19) **Europäisches Patentamt**

**European Patent Office**

**Office européen des brevets**

(11) **EP 1 073 896 B1**

(12) **EUROPÄISCHE PATENTSCHRIFT**

(45) Veröffentlichungstag und Bekanntmachung des Hinweises auf die Patenterteilung:
**14.09.2005 Patentblatt 2005/37**

(21) Anmeldenummer: **00907335.4**

(22) Anmeldetag: **24.02.2000**

(51) Int Cl.[7]: **G01N 33/14**, G01N 21/35

(86) Internationale Anmeldenummer:
**PCT/AT2000/000050**

(87) Internationale Veröffentlichungsnummer:
**WO 2000/050893 (31.08.2000 Gazette 2000/35)**

(54) **Verfahren zur spektroskopischen Bestimmung des Ethanolgehalts einer wässerigen Lösung**

Method for the spectroscopic determination of ethanol concentration in an aqueous sample

Procédé de détermination spectroscopique de la concentration d'éthanol dans un échantillon aqueux

(84) Benannte Vertragsstaaten:
**AT BE CH CY DE DK ES FI FR GB GR IE IT LI LU MC NL PT SE**

(30) Priorität: **25.02.1999 AT 32399**

(43) Veröffentlichungstag der Anmeldung:
**07.02.2001 Patentblatt 2001/06**

(73) Patentinhaber: **Anton Paar GmbH**
**8054 Graz (AT)**

(72) Erfinder:
• **BENES, Roman**
**A-8041 Graz (AT)**
• **PLESCHIUTSCHNIG, Josef**
**A-9150 Bleiburg (AT)**
• **REININGER, Franz**
**A-9500 Villach (AT)**
• **DEL BIANCO, Alessandro**
**A-9500 Villach (AT)**

(74) Vertreter: **Wildhack, Helmut et al**
**Patentanwälte**
**Dipl.-Ing. Dr. Helmut Wildhack**
**Dipl.-Ing. Dr.Gerhard Jellinek**
**Landstrasser Hauptstrasse 50**
**1030 Wien (AT)**

(56) Entgegenhaltungen:
**WO-A-93/17324          US-A- 5 679 955**

• **B.R. BUCHANAN, ET AL.: "Detection of Ethanol in Wines Using Optical-Fiber Measurements and Near-Infrared Analysis" APPLIED SPECTROSCOPY., Bd. 42, Nr. 6, 1988, Seiten 1106-1111, XP002140634 THE SOCIETY FOR APPLIED SPECTROSCOPY. BALTIMORE., US ISSN: 0003-7028**
• **CAVINATO A G ET AL: "NONINVASIVE METHOD FOR MONITORING ETHANOL IN FERMENTATION PROCESSES USING FIBER-OPTIC NEAR-INFRARED SPECTROSCOPY" ANALYTICAL CHEMISTRY,US,AMERICAN CHEMICAL SOCIETY. COLUMBUS, Bd. 62, Nr. 18, 15. September 1990 (1990-09-15), Seiten 1977-1982, XP000165593 ISSN: 0003-2700**
• **PATENT ABSTRACTS OF JAPAN vol. 1996, no. 11, 29. November 1996 (1996-11-29) & JP 08 178842 A (COSMO SOGO KENKYUSHO KK; COSMO OIL CO LTD), 12. Juli 1996 (1996-07-12)**
• **L.G. WEYER: "Near-Infrared Spectroscopy of Organic Substances" APPLIED SPECTROSCOPY REVIEWS., Bd. 21, Nr. 1/2, 1985, Seiten 1-43, XP002140635 MARCEL DEKKER INC.270 MADISON AVENUE. NEW YORK., US ISSN: 0570-4928**

**Beschreibung**

Die vorliegende Erfindung bezieht sich auf ein Verfahren zur spektroskopischen Bestimmung der Konzentration von Ethanol, in wässerigen Proben, insbesondere alkoholhaltigen Nahrungsmitteln, Heilmitteln, kosmetischen Produkten oder dgl.

Die spektroskopische Bestimmung der Konzentration von niedrigen Alkoholen, insbesondere Ethanol, in flüssigen Proben ist eine weit verbreitete Methode, wobei es durch die sogenannte Nah-Infrarot-Spektroskopie (NIR) gelingt, verschiedenste Parameter qualitativ und/oder quantitativ zu bestimmen. Der Wellenlängenbereich in der NIR-Spektroskopie erstreckt sich hiebei von etwa 700 nm bis etwa 2500 nm, wobei jedoch bei diesem Verfahren üblicherweise nicht Grundschwingungen, sondern Ober- und Kombinationsschwingungen gemessen werden. In diesem Wellenlängenbereich sind jedoch die Absorptionsfähigkeiten der zu untersuchenden Substanzen geringer und die Absorptionsbanden breiter und überlappen bzw. überlagern sich häufig, was eine Interpretation der Meßergebnisse schwierig und gegebenenfalls eindeutige und reproduzierbare Messungen unmöglich macht. Durch die Herstellung und Verfügbarkeit billiger Bauteile, welche für spektroskopische Untersuchungen in diesem Wellenbereich eingesetzt werden können, wie beispielsweise auf dem Gebiet der Glasoptik, der Halbleiterdetektoren und neuer Lichtquellen, sind jedoch die Möglichkeiten einer einfachen und raschen Untersuchung von Proben durch NIR-Spektroskopie bei Einsatz von einfachen Vorrichtungen zur Durchführung derartiger Verfahren immer weiter verbreitet.

Ein Verfahren der eingangs genannten Art zur spektroskopischen Bestimmung der Konzentration von niedrigen Alkoholen, insbesondere Ethanol, in flüssigen Proben ist beispielsweise aus der US-PS 5 679 955 bekannt geworden, wobei bei jeweils einer einzigen Wellenlänge im nahen Infrarotbereich Transmissionsmessungen vorgenommen werden. Der Wellenlängenbereich zur Durchführung der Messungen unter Berücksichtigung der zum weitaus überwiegenden Teil in einer alkoholhaltigen Probe enthaltenen Bestandteile Ethanol und Wasser ist bei diesem bekannten Verfahren derart gewählt, daß die Absorption durch das enthaltene Wasser gegenüber der Absorption durch den zusätzlich enthaltenen Alkohol überwiegt. Es werden eine Vielzahl von Kalibriermessungen bei jeweils einer Wellenlänge durchgeführt, worauf der Alkoholgehalt von weiteren Proben auf Basis der erhaltenen Kalibrierdaten bestimmt wird, wobei gemäß diesem bekannten Stand der Technik vorgeschlagen wird, unter Berücksichtigung der Absorptionskoeffizienten von Wasser und Ethanol die Messungen bei Wellenlängen von etwa 0,98 μm, etwa 1,3 μm und etwa 1,45 μm durchzuführen. Nachteilig bei diesem bekannten Verfahren ist die Tatsache, daß die Messungen somit bei Wellenlängen durchgeführt werden, in welchen der Absorptionskoeffizient von Wasser gegenüber demjenigen von Ethanol überwiegt, sodaß eine Bestimmung des Alkoholgehaltes nur mit einer entsprechend hohen Ungenauigkeit vorgenommen werden kann und weiters die Bestimmung des Alkoholgehalts gemäß der US-PS 5 679 955 auf Alkoholgehalte von weniger als 10 Vol.-% Alkohol beschränkt ist. Es ist somit das bekannte Verfahren im wesentlichen auf die Bestimmung der Alkoholkonzentration von Bieren beschränkt, während Getränke bzw. allgemein flüssige Proben mit einem höheren Alkoholgehalt, wie beispielsweise Weine, Brände, Heilmittel oder auch kosmetische Produkte, mit diesem Verfahren nicht untersucht werden können. Darüber hinaus ist eine Berücksichtigung von in den zu untersuchenden Proben enthaltenen Drittsubstanzen, welche das Meßergebnis teilweise beträchtlich beeinflussen können, bei Anwendung des bekannten Verfahrens nicht ohne weiteres möglich.

Die vorliegende Erfindung zielt darauf ab, ein Verfahren zur spektroskopischen Bestimmung der Konzentration von Ethanol, in wässerigen Proben zur Verfügung zu stellen, mit welchem über einen weiten Konzentrationsbereich mit erhöhter Genauigkeit eine Bestimmung der Ethanolkonzentration, in flüssigen Proben vorgenommen werden kann. Zur Lösung dieser Aufgabe ist das erfindungsgemäße Verfahren ausgehend von einem Verfahren der eingangs genannten Art im wesentlichen dadurch gekennzeichnet, daß die Absorption von Licht durch die zu untersuchende Probe bei wenigstens einer Wellenlänge im Bereich von 1170 nm bis 1200 nm, gemessen wird und daß aus der ermittelten Absorption mit Hilfe einer Kalibration die Ethanolkonzentration bestimmt wird. Dadurch, daß erfindungsgemäß die Absorption von Licht durch die zu untersuchende Probe bei wenigstens einer Wellenlänge im Bereich von 1170 nm bis 1200 nm gemessen wird, wird sichergestellt, daß die Messung in einem Wellenlängenbereich erfolgt, in welchem das Absorptionsspektrum des festzustellenden Ethanol, ein stark ausgeprägtes Absorptionsmaximum aufweist und sich somit leicht von dem Absorptionsspektrum des darüber hinaus zum überwiegenden Teil in der derartigen Probe befindlichen Wassers abtrennen bzw. unterscheiden läßt. Bei einer Messung der Absorption in dem erfindungsgemäß angegebenen Wellenlängenbereich läßt sich nachfolgend durch eine Kalibration, welche vorab unter Verwendung entweder einer künstlichen Probe, beispielsweise einer wäßrigen Alkoholmischung, oder einer realen Probe, beispielsweise Bier, Wein oder dgl., ermittelt wurde, unmittelbar der Alkoholgehalt der zu untersuchenden Probe feststellen.

Gemäß den erfindungsgemäßen Verfahren wird hiebei vorgeschlagen, daß die Absorption im Bereich von 1170 nm bis 1200 nm gemessen wird, da neben einem ausgeprägten Maximum des Absorptionskoeffizienten von Ethanol in diesem Wellenlängenbereich der Absorptionskoeffizient von Wasser nicht nur im wesentlichen konstant ist, sondern auch niedriger ist als der Absorptionskoeffizient von Ethanol. In diesem Zusammenhang wird darüber hinaus bevorzugt vorgeschlagen, daß die Messung der Absorption in Wellenlängenbereichen vorgenommen wird, in welchen der Absorptionskoeffizient von in der Probe enthaltenem Wasser ein im wesentlichen lineares, insbesondere konstantes, Verhalten aufweist, wodurch es insgesamt ermöglicht wird, den durch den Wassergehalt der zu untersuchenden Probe hervorgerufenen Anteil an der Gesamtabsorption in einfacher Weise zu ermitteln und bei der Auswertung zu berücksichtigen.

Wie oben bereits angedeutet, ist davon auszugehen, daß bei den zu untersuchenden Proben neben den Hauptbestandteilen niedriger Alkohol, insbesondere Ethanol, und Wasser teilweise Drittsubstanzen enthalten sind, welche die Bestimmung der Ethanolkonzentration in der zu untersuchenden Probe beeinflussen können. Neben den oben angeführten, erfindungsgemäß vorgeschlagenen Auswahlkriterien für den Wellenlängenbereich wird darüber hinaus bevorzugt vorgeschlagen, daß die Messung der Absorption in Wellenlängenbereichen vorgenommen wird, in welchen das Absorptionsverhalten von in der Probe enthaltenen Zusatzstoffen ein im wesentlichen lineares Verhalten aufweist, sodaß es in einfacher Weise gelingt, die durch derartige Zusatzstoffe zusätzlich hervorgerufenen Anteile bei der Messung der Absorption und bei der Bestimmung der Konzentration zu berücksichtigen. Hiebei kann besonders bevorzugt so vorgegangen werden, daß eine Auswahl der einzusetzenden Wellenlänge derart erfolgt, daß die Anteile von Zusatzstoffen bei der Absorption bei einer gewählten Wellenlänge einander im wesentlichen aufheben bzw. durch entsprechend vorgegebene Kalibrierfaktoren einfach berücksichtigbar sind.

Zur Erhöhung der Genauigkeit bei der Bestimmung der Alkoholkonzentration wird darüber hinaus vorgeschlagen, daß die Absorption von Licht bei wenigstens zwei Wellenlängen, insbesondere drei Wellenlängen, gemessen wird, wie dies einer weiteren bevorzugten Ausführungsform des erfindungsgemäßen Verfahrens entspricht. Derart kann durch Einsatz von wenigstens zwei, insbesondere von drei, Wellenlängen rasch eine Bestimmung einer Mehrzahl von Absorptionswerten der zu untersuchenden Probe erhalten werden, wobei durch einfache Auswerteverfahren eine Anpassung und Auswertung der gemessenen Werte erzielbar ist und in weiterer Folge ein einfacher Vergleich mit den vorher bestimmten Bezugswerten wenigstens einer Kalibriermessung vorgenommen werden kann. Für eine einfache Auswertung der bei mehreren Wellenlängen festgestellten Werte für die Absorption von Licht durch die zu untersuchende Probe wird erfindungsgemäß bevorzugt vorgeschlagen, daß zur Bestimmung der Alkoholkonzentration die für unterschiedliche Wellenlängen erhaltenen Absorptionswerte in einem linearen Näherungsverfahren, wie beispielsweise einer linearen Regression, einer multilinearen Regression, etc., ausgewertet werden und mit den in einer Kalibriermessung erhaltenen Bezugswerten verglichen werden, wobei bei Einsatz derartiger einfacher Näherungsverfahren auch ohne Zuhilfenahme von aufwendigen Auswerte- und Rechenverfahren das Auslangen gefunden werden kann.

Unter Berücksichtigung der Tatsache, daß das erfindungsgemäß vorgeschlagene Verfahren zur spektroskopischen Bestimmung der Ethanolkonzentration bei wenigstens einer Wellenlänge in einem Bereich durchgeführt wird, in welchem der Absorptionskoeffizient von Ethanol ein ausgeprägtes Maximum aufweist, während die Absorptionskoeffizienten von Wasser und gegebenenfalls enthaltenen Zusatzstoffen ein im wesentlichen lineares bzw. günstigerweise konstantes Verhalten aufweisen, gelingt es somit, die Alkoholkonzentration einer zu untersuchenden Probe in einem stark vergrößerten Konzentrationsbereich mit einer entsprechenden Genauigkeit durchzuführen, wobei in diesem Zusammenhang erfindungsgemäß besonders bevorzugt vorgeschlagen wird, daß die Ethanolkonzentration der zu untersuchenden Probe weniger als 60 % beträgt.

Zur weiteren Erhöhung der Genauigkeit bei der Feststellung der Alkoholkonzentration wird darüber hinaus vorgeschlagen, daß die Messung der Absorption bei konstanter Temperatur der zu untersuchenden Probe vorgenommen wird und/oder daß die gemessene Temperatur der zu untersuchenden Probe berücksichtigt wird, wie dies einer weiteren bevorzugten Durchführungsform des erfindungsgemäßen Verfahrens entspricht. Durch eine derartige konstante Temperatur bzw. Thermostatisierung der Probe läßt sich die Genauigkeit der zu bestimmenden Ethanolkonzentration weiter erhöhen bzw. es läßt sich bei Berücksichtigung der tatsächlichen Probentemperatur die Temperaturabhängigkeit der einzelnen Parameter ebenfalls berücksichtigen. Zur Erzielung einer entsprechenden Genauigkeit bei der Bestimmung der Ethanolkonzentration, welche günstigerweise höher als $\pm$ 0,2 Vol.-% und insbesondere bei $\pm$ 0,1 Vol.-% liegt, wird darüber hinaus bevorzugt vorgeschlagen, daß die Temperatur der Probe mit einer Genauigkeit von $\pm$ 0,5 °C, insbesondere $\pm$ 0,2 °C, eingestellt wird.

Die Erfindung wird nachfolgend anhand eines in der beiliegenden Zeichnung dargestellten Ausführungsbeispieles zur Durchführung des erfindungsgemäßen Verfahrens näher erläutert. In dieser zeigen:

Fig. 1 eine schematische Ansicht einer Vorrichtung zur Durchführung des erfindungsgemäßen Verfahrens;

Fig. 2 die Absorptionsspektren von Wasser und Ethanol für den Bereich der NIR-Spektroskopie; und

Fig. 3 die Absorptionsspektren von Wasser und Ethanol sowie einigen, beispielsweise in Wein enthaltenen Zusatzstoffen in einem eingeschränkten Wellenlängenbereich.

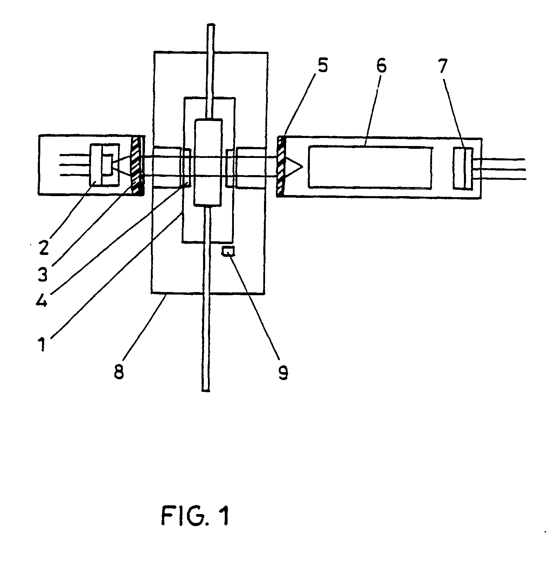

In der in Fig. 1 dargestellten Vorrichtung zur Durchführung eines Verfahrens zur spektroskopischen Bestimmung der Konzentration von niedrigen Alkoholen in flüssigen Proben ist mit 1 eine Küvette zur Aufnahme der zu untersuchenden Probe bezeichnet, wobei als Lichtquelle 2 beispielsweise eine Diode im nahen Infrarotbereich verwendet wird. Durch eine schematisch mit 3 bezeichnete Optik und ein Glasfenster 4 gelangt elektromagnetische Strahlung mit einem ausgesuchten Wellenlängenbereich auf die Küvette 1 mit der zu untersuchenden Probe und in weiterer Folge durch eine weitere, schematisch mit 5 bezeichnete Optik und ein dispersives Element 6 zu einem Detektor 7, welcher beispielsweise von einem Halbleiterdetektor-Array gebildet wird. Weiters ist in Fig. 1 eine Ummantelung 8 zur Thermostatisierung der zu untersuchenden Probe sowie mit 9 schematisch ein Sensor zur Temperaturbestimmung vorgesehen.

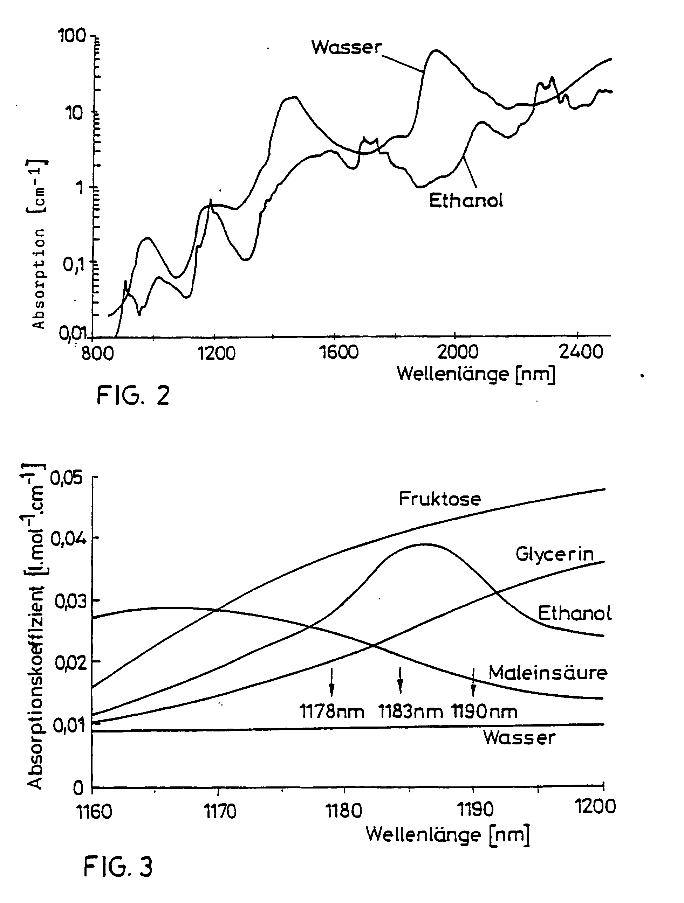

Aus den in Fig. 2 dargestellten Absorptionsspektren der Hauptbestandteile, Wasser und Ethanol, von alkoholhaltigen Proben ist ersichtlich, daß in einem Wellenlängenbereich von 1100 nm bis 1300 nm, und insbesondere von 1150 nm bis 1250 nm, der Absorptionskoeffizient von Ethanol einen ausgeprägten Peak bzw. ein Maximum aufweist, während in demselben Wellenlängenbereich der Absorptionskoeffizient von Wasser ein im wesentlichen konstantes bzw. zumindest lineares Absorptionsverhalten aufweist. Weiters ist ersichtlich, daß in einem Wellenlängenbereich von 1170 nm bis 1200 nm der Absorptionskoeffizient von Ethanol jenen von Wasser übersteigt, wobei dies noch deutlicher aus dem Diagramm gemäß Fig. 3 ersichtlich ist. In Fig. 3 ist besonders deutlich dargestellt, daß in dem genannten Wellenlängenbereich der Absorptionskoeffizient von Wasser nahezu konstant ist.

Neben den Absorptionsspektren von Wasser und Ethanol ist bei dem in Fig. 3 dargestellten Ausschnitt des Wellenlängenbereiches der Absorptionskoeffizient von Fructose, Glyzerin und Maleinsäure dargestellt, wobei diese übliche Inhaltsstoffe bzw. Drittsubstanzen in Wein darstellen. Aus Fig. 3 ist hiebei ersichtlich, daß in dem bevorzugten Wellenlängenbereich von 1170 nm bis 1200 nm neben dem charakteristischen Verlauf der Absorptionskoeffizienten von Ethanol als auch von Wasser die Absorptionskoeffizienten dieser wichtigsten, zusätzlichen Inhaltsstoffe im wesentlichen einen linearen und teilweise gegenläufigen Verlauf zeigen, sodaß es beispielsweise durch geeignete Wahl der Wellenlänge möglich wird, bei der Durchführung der Kalibriermessungen sowie bei der Durchführung der Absorptionsmessungen an den zu untersuchenden Proben eine Größe ΔA als Verhältnis der gemessenen Absorptionswerte insbesondere bei Verwendung von mehreren Wellenlängen zu bestimmen, sodaß sich die Absorptionsanteile insbesondere der weiteren Inhaltsstoffe leicht berücksichtigen lassen bzw. einander aufheben.

Die Bestimmung der Alkoholkonzentration bei Proben mit unterschiedlichem Alkoholgehalt bzw. unterschiedlicher Zusammensetzung werden darüber hinaus anhand der folgenden Ausführungsbeispiele näher erläutert.

Beispiel 1

Alkoholmessung in Wein:

Verwendete Wellenlängen: 1178 nm, 1183 nm, 1190 nm
Gemessene Absorptionen: $A_{1178}$, $A_{1183}$, $A_{1190}$

Es wird eine Größe ΔA wie folgt ermittelt:

$$\Delta A = A_{1183} - \left( \frac{7}{12} * A_{1178} + \frac{5}{12} * A_{1190} \right)$$

Diese Größe ΔA ist eine Funktion der Alkoholkonzentration (k):

$$\Delta A = a * k + b$$

wobei a und b aus vorangehenden Messungen bei einer Probe bekannter Konzentration ermittelte Kalibrierkonstanten sind und ungefähr $a=1.10^{-3}$, $b=1.10^{-4}$ für Wein betragen.
Erzielte Genauigkeit ± 0,1 Vol.-%

Es ist unmittelbar ersichtlich, daß durch ein einfaches lineares Näherungsverfahren direkt die Alkoholkonzentration der zu untersuchenden Probe bestimmt werden kann, wobei die zur Bestimmung der Größe ΔA angegebenen, einfachen Zusatzfaktoren im wesentlichen aus dem linearen Verhalten der Absorptionskoeffizienten der zusätzlichen Bestandteile in dem angegebenen Wellenlängenbereich resultieren und in einfachen Kalibriermessungen bestimmbar sind. Es ist weiters festzuhalten, daß die angegebenen Kalibrierkonstanten als Zusatzfaktoren zur Bestimmung der Größe ΔA hiebei in einem großen Konzentrationsbereich allgemein für Weine einheitlich einsetzbar sind, da selbst bei gegebenenfalls unterschiedlichen Anteilen, insbesondere der Zusatzstoffe, durch deren lineares Absorptionsverhalten diese Korrekturanteile immer ausreichend berücksichtigt sind.

Beispiel 2

Alkoholmessung in Bier:

Verwendete Wellenlängen: 1178 nm, 1183 nm, 1188 nm
Gemessene Absorptionen: $A_{1178}$, $A_{1183}$, $A_{1188}$

Es wird eine Größe ΔA wie folgt ermittelt:

$$\Delta A \;=\; A_{1183} \;-\; \left( \frac{1}{2} \;*\; A_{1178} \;+\; \frac{1}{2} \;*\; A_{1188} \right)$$

Diese Größe ΔA ist wiederum eine Funktion der Alkoholkonzentration (k):

$$\Delta A = a * k + b$$

wobei a und b Kalibrierkonstanten sind und ungefähr $a=8.10^{-4}$, $b=1.10^{-4}$ betragen.
Erzielte Genauigkeit $\pm$ 0,1 Vol.-%

Beispiel 3

Alkoholmessung in Getränken mit hohem Alkoholgehalt (Schnäpsen):

Verwendete Wellenlängen: 1178 nm, 1184 nm, 1188 nm
Gemessene Absorptionen: $A_{1178}$, $A_{1184}$, $A_{1188}$

Es wird eine Größe ΔA wie folgt ermittelt:

$$\Delta A = A_{1184} - (0,6 * A_{1178} + 0,4 * A_{1188})$$

Diese Größe ΔA ist wiederum eine lineare Funktion der Alkoholkonzentration (k):

$$\Delta A = a * k + b$$

wobei a und b Kalibrierkonstanten sind und ungefähr $a=9.10^{-4}$, $b=8.10^{-4}$ für Schnäpse betragen.
Gültigkeitsbereich: 25 Vol.-% bis 50 Vol.-%
Erzielte Genauigkeit $\pm$ 0,1 Vol.-%

Zu den Beispielen 2 und 3 ist festzuhalten, daß ähnlich wie bei der Darstellung in Fig. 3 für die Zusatzstoffe in Wein auch allgemein für Biere und hochalkoholische Getränke eine geringe Anzahl von Zusatzstoffe ermittelbar ist, wobei auch diese in dem angegebenen Wellenlängenbereich ein im wesentlichen lineares Verhalten aufweisen, sodaß wiederum mit einfachen Korrekturgrößen bzw. Kalibrierkonstanten zur genauen und exakten Ermittlung des Alkoholgehalts das Auslangen gefunden werden kann und derart eine einfache Bestimmung der Alkoholkonzentration über weite Konzentrationsbereiche ermöglicht wird.

In ähnlicher Weise wie bei den vorangegangenen Ausführungsbeispielen läßt sich der Alkoholgehalt beispielsweise in Heilmitteln unter Berücksichtigung der neben Wasser und Alkohol enthaltenen Zusatzstoffe als auch in alkoholhaltigen, kosmetischen Produkten ermitteln.

**Patentansprüche**

1.  Verfahren zur spektroskopischen Bestimmung der Konzentration von Ethanol in flüssigen Proben aus der Gruppe alkoholhaltige Nahrungsmittel, Heilmittel, kosmetische Produkte, wobei die Absorption von Licht durch die zu untersuchende Probe bei wenigstens einer Wellenlänge im Bereich von 1150 nm bis 1250 nm, gemessen wird und aus der ermittelten Absorption mit Hilfe einer Kalibration die Alkoholkonzentration bestimmt wird, **dadurch gekennzeichnet, dass**

- die Absorption in dem Wellenlängenbereich von 1170 nm bis 1200 nm gemessen wird, in welchem der Absorptionskoeffzient des in der Probe enthaltenen Wassers niedriger ist als der Absorptionskoeffizient des Ethanols und einen im wesentlichen linearen oder konstanten Verlauf aufweist und
- das Absorptionsspektrum des Ethanols ein stark ausgeprägtes Absorptionsmaximum aufweist und sich somit von dem Absorptionsspektrum des zum überwiegenden Teil in der Probe befindlichen Wassers abtrennen bzw. unterscheiden lässt.

2. Verfahren nach Anspruch 1, **dadurch gekennzeichnet, dass** die Absorption innerhalb eines Wellenlängenbereiches von 1178 nm bis 1190 nm, insbesondere von 1178 nm bis 1188 nm, gemessen wird.

3. Verfahren nach Anspruch 1 oder 2, **dadurch gekennzeichnet, dass**

- die Absorption von Licht bei wenigstens zwei Wellenlängen, insbesondere bei drei Wellenlängen, innerhalb der dort genannten Bereiche gemessen wird, und
- zur Bestimmung der Ethanolkonzentration die für die zwei, insbesondere drei, unterschiedlichen Wellenlängen erhaltenen Absorptionswerte mittels eines linearen Näherungsverfahrens, vorzugsweise mittels linearer oder multilinearer Regression, ausgewertet werden und mit den in einer Kalibriermessung erhaltenen Bezugswerten verglichen werden.

4. Verfahren nach einem der Ansprüche 1 bis 3, **dadurch gekennzeichnet, dass** die Messung der Absorption in einem Wellenlängenbereich vorgenommen wird, in welchem das Absorptionsverhalten der in der Probe enthaltenen Zusatzstoffe ein im wesentlichen lineares Verhalten aufweist.

5. Verfahren nach einem der Ansprüche 1 bis 4, **dadurch gekennzeichnet, dass** die Alkoholkonzentration der zu untersuchenden Probe auf weniger als 60 % eingestellt wird.

6. Verfahren nach einem der Ansprüche 1 bis 5, **dadurch gekennzeichnet, dass**

- die Messung der Absorption bei konstanter Temperatur der zu untersuchenden Probe vorgenommen wird und/ oder
- die gemessene Temperatur der zu untersuchenden Probe berücksichtigt wird.

7. Verfahren nach Anspruch 6, **dadurch gekennzeichnet, dass** die Temperatur der Probe mit einer Genauigkeit von ± 0,5°C, insbesondere ± 0,2°C, eingestellt wird.

**Claims**

1. Process for the spectroscopic determination of the concentration of ethanol in liquid samples from the group of alcohol-containing foodstuffs, therapeutic agents, cosmetic products, wherein the absorption of light by the sample to be investigated is measured at at least one wavelength in the range of 1150 nm to 1250 nm, and the alcohol concentration is determined with the aid of a calibration from the absorption found, **characterised in that**

- the absorption is measured in the wavelength range of 1170 nm to 1200 nm, in which the absorption coefficient of the water contained in the sample is lower than the absorption coefficient of the ethanol and has a substantially linear or constant curve, and

- the absorption spectrum of the ethanol has a very distinct absorption maximum and can thus be separated or differentiated from the absorption spectrum of the water that is present as the major portion in the sample.

2. Process according to claim 1, **characterised in that** the absorption is measured within a wavelength range of 1178 nm to 1190 nm, particularly of 1178 nm to 1188 nm.

3. Process according to claim 1 or 2, **characterised in that**

- the absorption of light is measured at at least two wavelengths, particularly at three wavelengths, within the ranges mentioned there, and

- to determine the ethanol concentration, the absorption values obtained for two, particularly three, different wavelengths are evaluated by a linear approximation method, preferably by linear or multi-linear regression, and compared with the reference values obtained in a calibration measurement.

**4.** Process according to one of claims 1 to 3, **characterised in that** the measurement of the absorption is performed in a wavelength range in which the absorption behaviour of the additives contained in the sample exhibits substantially linear behaviour.

**5.** Process according to one of claims 1 to 4, **characterised in that** the alcohol concentration of the sample to be investigated is adjusted to less than 60%.

**6.** Process according to one of claims 1 to 5, **characterised in that**

- the measurement of the absorption is performed at a constant temperature of the sample to be investigated and/or
- the measured temperature of the sample to be investigated is taken into account.

**7.** Process according to claim 6, **characterised in that** the temperature of the sample is adjusted with an accuracy of $\pm$ 0.5°C, particularly $\pm$ 0.2°C.

**Revendications**

**1.** Procédé de détermination spectroscopique de la concentration d'éthanol dans des échantillons aqueux du groupe des aliments d'une teneur en alcool, des médicament, des produits cosmétiques, dans lequel l'absorption de la lumière par l'échantillon à examiner à au moins une longueur d'onde dans la gamme de 1150 nm à 1250 nm est mesurée, et la concentration d'alcool est déterminée par l'absorption déterminée au moyens d'un calibrage, **caractérisé en ce, que**

- l'absorption est mesurée dans la gamme d'ondes de 1170 nm à 1200 nm, dans laquelle le coefficient d'absorption de l'eau contenu dans l'échantillon est plus bas que le coefficient d'absorption de l'éthanol et montre un cours essentiellement linéaire ou constant, et
- le spectre d'absorption de l'éthanol comprends un maximum d'absorption fortement prononcé et peut donc être séparée ou distinguée du spectre d'absorption de l'eau étant dans l'échantillon pour une part prépondérante.

**2.** Procédé selon la revendication 1, **caractérisé en ce que** l'absorption est mesurée au dedans de la gamme d'ondes de 1178 nm à 1190 nm, particulièrement de 1178 nm à 1188 nm.

**3.** Procédé selon la revendication 1 ou 2, **caractérisé en ce que**

- l'absorption de la lumière est mesurée à au moins deux longueurs d'onde, particulièrement à trois longueurs d'onde, au dedans des gammes y mentionnées, et que
- pour déterminer la concentration d'éthanol, les valeurs d'absorption obtenus pour les deux, particulièrement trois, longueurs d'onde différentes sont évalués au moyens d'une méthode approximative linéaire, de préférence au moyens d'une régression linéaire ou multilinéaire, et sont comparés avec les valeurs de référence obtenus dans une mesure de calibrage.

**4.** Procédé selon une quelconque des revendications 1 à 3, **caractérisé en ce que** le mesurage de l'absorption est effectué dans une gamme d'ondes, dans laquelle l'attitude d'absorption des additifs contenus dans l'échantillon montre une attitude essentiellement linéaire.

**5.** Procédé selon une quelconque des revendications 1 à 4, **caractérisé en ce que** la concentration d'alcool de l'échantillon à examiner est ajustée à moins de 60 %.

6. Procédé selon une quelconque des revendications 1 à 5, **caractérisé en ce que**

   - le mesurage de l'absorption est effectué à une température constante de l'échantillon à examiner et/ou
   - la température mesurée de l'échantillon à examiner est pris en considération.

7. Procédé selon la revendication 6, **caractérisé en ce que** la température de l'échantillon est ajustée avec une exactitude de $\pm$ 0,5°C, particulièrement de $\pm$ 0,2°C.

FIG. 1

FIG. 2

FIG. 3